# EUROPEAN PATENT APPLICATION

(11) **EP 4 578 952 A1**
(43) Date of publication of application: **02.07.2025**
(21) Application number: 23857313.3
(22) Date of filing: 21.08.2023
(51) Int. Cl.: C12N 15/88, C12N 5/071, C12N 5/10, C12N 15/85, C12N 15/86

(54) **METHOD FOR INTRODUCING NUCLEIC ACID INTO CELLS AND APPLICATION THEREOF**

(30) Priority: 22.08.2022 JP 2022131790
(71) Applicant: FUJIFILM Corporation, Tokyo 106-8620 (JP)
(72) Inventor: NISHINO, Masafumi, Ashigarakami-gun, Kanagawa 258-8577 (JP); SATO, Ryota, Ashigarakami-gun, Kanagawa 258-8577 (JP); MURAGUCHI, Taichi, Ashigarakami-gun, Kanagawa 258-8577 (JP); MORIYA, Kai, Ashigarakami-gun, Kanagawa 258-8577 (JP)
(74) Representative: Hoffmann Eitle
(86) International application number: PCT/JP2023/029939
(87) International publication number: WO 2024/043202

(57) **Abstract**

An object of the present invention is to provide a method of introducing a nucleic acid into a cell, in which a high concentration of a nucleic acid can be efficiently introduced into a cell having high density as a complex of the nucleic acid and a cationic polymer; a method of producing a cell into which a nucleic acid has been introduced and a method of producing a useful substance; and a composition containing a cationic polymer and a nucleic acid, and a cell culture product containing the composition and a cell. According to the present invention, there is provided a method of introducing a nucleic acid into a cell, the method including (a) a complex formation step of mixing a cationic polymer with a nucleic acid to obtain a solution of a complex of the cationic polymer and the nucleic acid, in which in the complex formation step, a concentration of the nucleic acid is 100 to 1,000 µg/mL and an electrical conductivity of a complex solution after the mixing is 1.0 to 7.0 mS/cm, and (b) a transfection step of adding the complex solution obtained in the step (a) to a cell suspension.

## Description

### Technical Field

The present invention relates to a method of introducing a nucleic acid into a cell, the method including transfecting a cell with a complex of a cationic polymer and a nucleic acid. The present invention further relates to a method of producing a cell into which a nucleic acid has been introduced, and a method of producing a useful substance. The present invention further relates to a complex containing a cationic polymer and a nucleic acid, and a cell culture product containing the complex and a cell.

### Background Art

To produce a gene therapy agent such as an adeno-associated virus (AAV) vector, a cell is transiently transfected with a gene. Examples of the method of producing an AAV vector include a method of transiently introducing a plasmid for AAV production, which is a nucleic acid, into a human embryonic kidney cells (HEK) 293 cell line to produce an AAV vector.

The introduction of nucleic acids into cells is essential not only for medical applications such as antibody formulations and gene therapy but also for basic research in molecular cell biology. For example, as a method of introducing a gene into a cell, a transfection method using a viral vector and a non-viral transfection method are known.

A transfection method using a virus vector results in a high level of gene introduction. The virus vector is an efficient gene introduction carrier, but the use thereof is limited because of induction of an immune response and virus-related pathogenicity.

The non-viral transfection method has no problems of the transfection method using a viral vector, and is advantageous in the production of a pharmaceutical drug from a nucleic acid. According to the non-viral transfection method, a product can be manufactured at an acceptable price with high reproducibility.

Many non-viral transfection methods are known, and examples of the main method thereof include a method using a cationic lipid and/or a cationic polymer. Currently, a series of non-viral transfection reagents containing cationic lipids or cationic polymers as a main component are commercially available and used.

Polyethyleneimine (hereinafter, PEI), which is a representative example of cationic polymers, is a low-cost, non-viral, and non-liposomal transfection reagent, and is known as one of the media with high cost-effectiveness. PEI forms a complex with a nucleic acid, thereby a gene can be efficiently transfected with low toxicity to cells. This PEI/nucleic acid complex is taken into the cell by endocytosis. PEI has a high pH buffer capacity, protects the incorporated DNA from decomposition in a lysosome, and efficiently releases the DNA from the lysosome to cytoplasm. As described above, PEI is useful as a transfection reagent and is used for transfection of DNA into mammalian cells.

For example, Patent Document 1 describes a method of additionally adding free PEI during the formation of a complex of PEI and a nucleic acid. Patent Document 2 describes transfecting a nucleic acid using PEI grafted with polyethylene glycol. Patent Document 3 describes transfecting a nucleic acid using PEI modified with polyethylene glycol. Patent Document 4 describes that the pH during the formation of a complex of PEI and a nucleic acid is defined under acidic conditions of 3.5 to 4.5. Non-Patent Documents 1 and 2 also describe that PEI is used for transfection.

### Prior Art Documents

### Patent Documents

Patent Document 1: JP2020-524498A
Patent Document 2: JP2021-106580A
Patent Document 3: JP2002-506441A
Patent Document 4: JP2011-24493A

### Non-Patent Documents

Non-Patent Document 1: Ogris, M. et al., The size of DNA/transferring-PEI complexes is an important factor for gene expression in cultured cells. Gene Ther. 5 (1998) 1425-1433
Non-Patent Document 2: Gonzalez-Dominguez et al (2019) Impact of physicochemical properties of DNA/PEI complexes on transient transfection of mammalian cells, New Biotechnology, Volume 49, 25 March 2019, pages 88-97

### SUMMARY OF THE INVENTION

### Object to be solved by the invention

In the gene introduction step in the related art, in the step of forming a complex of a nucleic acid and PEI, it is necessary to set a concentration of the complex to an appropriate range from the viewpoint of controlling a particle diameter of the complex.

On the other hand, as a result of intensive studies to produce a large amount of a target substance, it has been found that it is necessary to introduce a high concentration of a nucleic acid into cells. However, for this purpose, it is necessary to increase the amount of the solvent from the viewpoint of keeping the concentration of the complex in the step of forming the complex within an appropriate range. This is because, in a case where the concentration of the complex is not maintained within an appropriate range, the gene introduction efficiency is significantly reduced, and it is not possible to achieve the production of a large amount of a target protein.

An object to be achieved of the present invention is to provide a method of introducing a nucleic acid into a cell, in which a high concentration of the nucleic acid can be efficiently introduced as a complex of the nucleic acid and a cationic polymer. Another object to be achieved of the present invention is to provide a method of producing a cell into which a nucleic acid has been introduced, and a method of producing a useful substance, which use the above-described method of introducing a nucleic acid into a cell. Another object to be achieved of the present invention is to provide a composition containing a cationic polymer and a nucleic acid, and a cell culture product containing the composition and a cell, which are related to the above-described method.

### Means for solving the object

As a result of intensive studies to achieve the above-described objects, the present inventors have found that the above objects can be achieved by setting the electrical conductivity of a complex solution to 1.0 to 7.0 mS/cm in the formation of the complex in which a cationic polymer and a nucleic acid are mixed to obtain a solution of the complex of the cationic polymer and the nucleic acid. The present invention has been completed based on the above findings.

According to the present invention, the following inventions are provided.
<1> A method of introducing a nucleic acid into a cell, the method comprising (a) a complex formation step of mixing a cationic polymer with a nucleic acid to obtain a solution of a complex of the cationic polymer and the nucleic acid, in which in the complex formation step, a concentration of the nucleic acid is 100 to 1,000 µg/mL and an electrical conductivity of the complex solution after the mixing is 1.0 to 7.0 mS/cm, and (b) a transfection step of adding the complex solution obtained in the step (a) to a cell suspension.
<2> The method according to <1>, in which a period in which the electrical conductivity is 1.0 to 7.0 mS/cm is any one of 0 minutes to 10 minutes after a start of mixing the cationic polymer with the nucleic acid.
<3> The method according to <1> or <2>, in which the concentration of the nucleic acid is 110 µg/mL to 300 µg/mL.
<4> The method according to any one of <1> to <3>, in which a mass ratio between the cationic polymer and the nucleic acid is 8:1 to 1:1.
<5> The method according to any one of <1> to <4>, in which a cell density of the cell suspension in a case of being brought into contact with the complex solution is 1 × 10⁵ cells/mL to 5 × 10⁸ cells/mL.
<6> The method according to any one of <1> to <5>, in which a cell density of the cell suspension in a case of being brought into contact with the complex solution is 5 × 10⁶ cells/mL to 3 × 10⁷ cells/mL.
<7> The method according to any one of <1> to <6>, in which a total amount of the nucleic acid used for forming the complex is 0.1 to 1 µg per 1 million cells.
<8> The method according to any one of <1> to <7>, in which the electrical conductivity of the complex solution is 3.0 to 5.0 mS/cm.
<9> The method according to any one of <1> to <8>, in which an average particle diameter of the complex is 200 nm to 1,200 nm.
<10> The method according to any one of <1> to <9>, in which the complex formation step (a) includes, before the step (b), mixing the cationic polymer with the nucleic acid and then allowing the solution of the complex to stand for 10 seconds to 4 hours.
<11> The method according to any one of <1> to <10>, in which a volume ratio between the complex solution and the cell suspension is 1:2 to 1:500.
<12> The method according to any one of <1> to <11>, in which the cationic polymer is a polyamine.
<13> The method according to any one of <1> to <12>, in which the cationic polymer is polyethyleneimine.
<14> The method according to any one of <1> to <13>, in which the nucleic acid is a plasmid.
<15> The method according to any one of <1> to <14>, in which the cell is an animal cell.
<16> The method according to any one of <1> to <14>, in which the cell is an HEK cell.
<17> A method of producing a cell into which a nucleic acid has been introduced, the method comprising the method according to any one of <1> to <16>.
<18> A production method for a useful substance, comprising a step of introducing a nucleic acid into a cell by the method according to any one of <1> to <16>, and a step of culturing the cell to produce a useful substance.
<19> The method according to <18>, in which the useful substance is a virus vector.
<20> The method according to <19>, in which the virus vector is an active pharmaceutical ingredient.
<21> A composition comprising a cationic polymer and a nucleic acid, in which a concentration of the nucleic acid is 100 to 1,000 µg/mL, and an electrical conductivity is 1.0 to 7.0 mS/cm.
<22> The composition according to <21>, in which the composition is a composition for gene introduction.
<23> A cell culture product comprising the composition according to <21> or <22>, and cells having a cell density of 1 × 10⁵ cells/mL to 5 × 10⁸ cells/mL.

### Effect of the invention

According to the present invention, a high concentration of a nucleic acid can be efficiently introduced into cells as a complex of the nucleic acid and a cationic polymer. According to the present invention, it is possible to form a complex of a nucleic acid and a cationic polymer at a high concentration, and it is possible to avoid a substantial reduction in culture scale due to an increase in the density of cells into which the complex is introduced.

### EMBODIMENTS FOR CARRYING OUT THE INVENTION

Hereinafter, an example of the embodiment of the present disclosure will be described. However, the present disclosure is not limited to the following embodiments, and can be implemented with appropriate changes within the scope of the object of the present disclosure. In the present specification, a numerical range indicated using "to" means a range including numerical values described before and after "to" as a minimum value and a maximum value, respectively.

The present invention relates to a method of introducing a nucleic acid into a cell, including (a) a complex formation step of mixing a cationic polymer with a nucleic acid to obtain a solution of a complex of the cationic polymer and the nucleic acid, in which in the complex formation step, a concentration of the nucleic acid is 100 to 1,000 µg/mL and an electrical conductivity of a complex solution after the mixing is 1.0 to 7.0 mS/cm, and (b) a transfection step of adding the complex solution obtained in the step (a) to a cell suspension.

A first feature of the present invention is that the concentration of the nucleic acid in the complex formation step is high, that is, 100 to 1,000 µg/mL.A second feature of the present invention is that the electrical conductivity of the complex solution after mixing is 1.0 to 7.0 mS/cm.

The present invention is not limited, but in the present invention, a stabilizer (polyethylene glycol or the like) or an enhancer (addition of a cationic polymer such as PEI later) is not necessary. In addition, the present invention is not limited, but in the present invention, it is not necessary to set the pH during the formation of the complex of PEI and the nucleic acid to acidic conditions of 3.5 to 4.5.

In the present invention, even in a case where the concentration of the nucleic acid in the complex formation step is a high concentration as 100 to 1,000 µg/mL, the increase in the particle diameter of the complex can be controlled by setting the electrical conductivity of the complex solution within a range of 1.0 to 7.0 mS/cm, thereby the complex of the nucleic acid and the cationic polymer can be efficiently introduced into the cells.

In the present invention, a cationic polymer and a nucleic acid are mixed to obtain a solution of a complex of the cationic polymer and the nucleic acid.

The cationic polymer is not particularly limited, and for example, one type selected from the group consisting of chitosan, poly-L-lysine (pLL), polyamine (PA), polyalkyleneimine (PAI), polyethyleneimine (PEI), poly[a-(amino butyl)-L-glycolic acid], polyamidoamine, poly(2-dimethylamino)ethyl methacrylate, polyhistidine, polyarginine, poly(4-vinylpyridine), poly(vinylamine), and poly(4-vinyl-N-alkylpyridinium halide), or a combination thereof can be used.

As the cationic polymer, polyamine is preferable, and polyethyleneimine (hereinafter, also referred to as PEI) is particularly preferable. As the cationic polymer, it is preferable to use a non-lipid cationic polymer.

The PEI may be linear PEI or branched PEI, but is preferably linear PEI.

The molecular weight of PEI is preferably 10,000 to 800,000 Da, more preferably 10,000 to 50,000 Da, still more preferably 10,000 to 27,000 Da, and particularly preferably about 25,000 Da, in consideration of the transfection efficiency. As an example, linear PEI having a molecular weight of about 25,000 Da can be used. PEI is commercially available and can be easily obtained. For example, PEIpro (101000033) manufactured by Polyplus can be preferably used.

The nucleic acid refers to a chain-like polynucleotide in which a nucleotide consisting of a purine or pyrimidine base, a sugar, and a phosphate is a basic unit, and the phosphate forms a diester bond between the 3' and 5' carbon atoms of the sugar between each of the nucleotides to polymerize. Due to the difference in sugar, nucleic acids are roughly classified into deoxyribonucleic acid (DNA) having a sugar moiety of deoxyribose and ribonucleic acid (RNA) having a sugar moiety of ribose. The nucleic acid may be any one of a gene, DNA (linear DNA or circular DNA), RNA, an oligonucleotide, or a polynucleotide.

In the present specification, the nucleic acid is used interchangeably with a gene, DNA, RNA, an oligonucleotide, and a polynucleotide.

An example of the nucleic acid in the present invention is a plasmid.

The concentration of the nucleic acid in the complex formation step is 100 µg/mL to 1,000 µg/mL, preferably 110 µg/mL to 1,000 µg/mL, more preferably 110 µg/mL to 500 µg/mL, still more preferably 110 µg/mL to 300 µg/mL, particularly preferably 110 µg/mL to 200 µg/mL, and most preferably 110 µg/mL to 150 µg/mL.

The concentration of the nucleic acid can be measured with a microspectrophotometer Nanodrop (trademark) 2000c (ND-2000c, Thermo Fisher Scientific).

In the complex formation step, the mass ratio between the cationic polymer and the nucleic acid is preferably 8:1 to 1:1, more preferably 5:1 to 1:1, and still more preferably 3:1 to 1:1.

In the present invention, the electrical conductivity of the complex solution is 1.0 to 7.0 mS/cm. The period in which the electrical conductivity of the complex solution is 1.0 to 7.0 mS/cm may be any period of 0 minutes to 10 minutes after the start of the mixing of the cationic polymer with the nucleic acid.

The electrical conductivity of the complex solution is preferably 2.0 mS/cm to 6.0 mS/cm and more preferably 3.0 mS/cm to 5.0 mS/cm.

The electrical conductivity of the complex solution can be measured by using the electrical conductivity meter, inserting an electrode of an electrical conductivity meter into each sample in which the temperature is controlled to 25°C, and measuring the electrical conductivity.

The average particle diameter of the complex formed in the complex formation step is preferably 200 nm to 1,200 nm, more preferably 300 nm to 1,200 nm, still more preferably 400 nm to 1,100 nm, and particularly preferably 500 nm to 1,000 nm.

The average particle diameter of the complex can be measured by a dynamic light scattering method as a standard of scattering intensity. The analysis method is a cumulant method, and the viscosity and the refractive index can be measured by using values actually measured by a viscometer and a refractometer.

In the complex formation step (a), preferably, the cationic polymer and the nucleic acid may be mixed and then the complex solution may be allowed to stand for 10 seconds to 4 hours, more preferably allowed to stand for 1 minute to 60 minutes, and still more preferably allowed to stand for 10 minutes to 30 minutes before the step (b). In addition, the temperature during the standing is preferably 15°C to 35°C, more preferably 20°C to 30°C, and particularly preferably 22°C to 28°C.

In the present invention, a transfection step of adding the complex solution obtained in the step (a) to a cell suspension is performed. Transfection means introducing a nucleic acid into a cell.

The cell density of the cell suspension in a case of being brought into contact with the complex solution is not particularly limited, but it is preferably 1 × 10⁵ cells/mL to 5 × 10⁸ cells/mL, more preferably 1 × 10⁵ cells/mL to 1 × 10⁸ cells/mL, still more preferably 5 × 10⁵ cells/mL to 5 × 10⁷ cells/mL, particularly preferably 1 × 10⁶ cells/mL to 3 × 10⁷ cells/mL, and most preferably 5 × 10⁶ cells/mL to 3 × 10⁷ cells/mL.

The cell density can be determined using Vi-cell (trademark) XR (manufactured by Beckman Coulter, Inc.).

The total amount of the nucleic acid used for forming the complex is not particularly limited, but it is preferably 0.1 to 1 µg per 1 million cells, more preferably 0.2 to 0.9 µg per 1 million cells, still more preferably 0.3 to 0.8 µg per 1 million cells, and particularly preferably 0.4 to 0.7 µg per 1 million cells.

The volume ratio between the complex solution and the cell suspension is not particularly limited, but it is preferably 1:2 to 1:500, more preferably 1:5 to 1:300, particularly preferably 1:10 to 1:200, and most preferably 1:20 to 1:200. The lower limit of the volume ratio between the complex solution and the cell suspension may be 1:5, 1:10, or 1:20. The upper limit of the volume ratio between the complex solution and the cell suspension may be 1:300 or 1:200.

The cell used in the present invention may be any one of an isolated cell, a cell included in an isolated living body-derived tissue, or a cultured cell. In a case where the cell is an isolated cell or a cultured cell, the cell may be any of an adherent cell or a floating cell.

As the cell, an animal cell is preferable. The animal cell is not particularly limited, but is preferably a mammalian cell or an insect cell, and more preferably a mammalian cell. Examples of the mammalian cell include a human cell, a mouse cell, a rat cell, a monkey cell, and a hamster cell, but the cells are not particularly limited. The human cell can be preferably used. Examples of the cell include mouse myeloma (NSO) cell lines, Chinese hamster ovary (CHO) cell lines, HT1080, H9, HepG2, MCF7, MDBK Jurkat, NIH3T3, PC12, a baby hamster kidney (BHK) cell, VERO, SP2/0, YB2/0, Y0, C127, an L cell, COS (for example, COS1 and COS7), QC1-3, human embryonic kidney (HEK) cells (for example, HEK293 and the like), VERO, PER. C6, HeLa, EB1, EB2, EB3, and oncolytic or hybridoma cell lines. The cell is preferably a HEK cell or a CHO cell, and more preferably a HEK293 cell.

According to the present invention, there is provided a method of producing a cell into which a nucleic acid has been introduced, the method including a method of introducing a nucleic acid into a cell according to the present invention.

According to the present invention, there is further provided a method of producing a useful substance, including a step of introducing a nucleic acid into a cell by the method according to the embodiment of the present invention and a step of culturing the cell to produce a useful substance.

The useful substance is not particularly limited, and examples thereof include a virus vector and a protein. The useful substance is preferably a viral vector. The protein or virus vector, which is produced, may be used, for example, as an active pharmaceutical ingredient.

The virus means a virus that is produced by introducing a nucleic acid into a cell. Examples of the virus include an adeno-associated virus, a lentivirus, a baculovirus, and a retrovirus, and among these, an adeno-associated virus is preferable.

Adeno-associated virus (AAV) refers to a small, incompletely replicative, non-enveloped virus containing single-stranded DNA having about 4,700 bases, from the family of Parvoviridae and Dependoparvovirus. It is known that there are more than 100 serum types in AAV, and that the host range and the characteristics of the virus differ depending on the difference in the serum types. Serum type 2 (AAV2) is one of the serum types that have been widely studied for a long time, and it is known that the host range is very wide. The serum type 1 (AAV1), the serum type 5 (AAV5), and the serum type 6 (AAV6) are serum types having higher tissue directivity. It is said that AAV1 has high gene introduction efficiency into muscle, liver, airway, central nervous system, and the like, AAV5 has high gene introduction efficiency into central nervous system, liver, retina, and the like, and AAV6 has high gene introduction efficiency into heart, muscle, liver, and the like. In the present invention, the serum type 2 or the serum type 5 is preferably used. The serum type 5 is particularly preferable.

The adeno-associated virus gene refers to a gene composed of one or a plurality of nucleic acid sequences derived from the serum types of one or a plurality of adeno-associated viruses. The adeno-associated virus gene is preferably a gene involved in replication and packaging of AAV and a gene encoding an AAV constituent protein.

AAV is a non-enveloped virus that proliferates in the presence of helper viruses such as adenovirus and herpesvirus. In the preparation of AAV to be used for gene therapy or nucleic acid introduction, classically, AAV replication has been performed by co-infecting host cells with an adenovirus. In addition, a gene responsible for the helper action of adenovirus has been clarified, and a plasmid bearing this gene has also been used. For example, a plasmid containing a Rep gene and a Cap gene, an adenovirus helper plasmid, and a plasmid containing a gene for treatment or prevention are simultaneously transfected into cells, and can thus be packaged as recombinant AAV (rAAV).

The Rep gene and the Cap gene encode proteins involved in the replication and packaging of the virion. In the wild type, the Rep gene is expressed from the P5 promoter and the p19 promoter. The Cap region expresses VP1, VP2, and VP3. Examples of the promoter that is naturally carried by the Cap gene can include a p40 promoter.

The adeno-associated virus gene (such as a Rep gene and a Cap gene) may be a wild-type gene, but a gene in which modifications such as substitution, deletion, insertion, or addition of a base have been made to the wild-type gene may be used as long as it exhibits an inherent function.

In a case where the Rep gene and the Cap gene are introduced into a cell, a vector containing the Rep gene and the Cap gene can be introduced into the cell. The Rep gene means a region of the AAV genome encoding a virus replication protein that is known to those skilled in the art and that is collectively required for replication of a virus genome, or its functional homolog, for example, a human herpesvirus 6 (HHV-6) Rep gene (mediating AAV-2 DNA replication is known). The Cap gene means a region in the AAV genome encoding a capsid protein of a virus known to those skilled in the art.

As the vector containing the Rep gene and the Cap gene, for example, a plasmid, a nucleic acid sequence derived from a virus, or an artificially designed nucleic acid can be used, and a plasmid is preferable.

In the present invention, a gene for treatment or prevention may be introduced into cells.

As the gene for treatment or prevention, a gene that is incomplete or lost in the genome of the target cell, a gene encoding non-natural protein having a desired biological or therapeutic effect (for example, antiviral function), or the like can be used, but the gene is not particularly limited. Specific examples of the gene for treatment or prevention include a gene used for treatment or prevention of inflammatory diseases, autoimmunity, chronic and infectious diseases (including disorders such as AIDS, cancer, nervous system diseases, cardiovascular diseases, and hypercholesterolemia), various blood diseases such as anemia and hemophilia, or gene deficiency (for example, cystic fibrosis, Gaucher's disease, adenosine deaminase (ADA) deficiency, emphysema, and the like).

The gene for treatment or prevention may be several antisense oligonucleotides (for example, a short-chain oligonucleotide complementary to a sequence around a translation initiation site (AUG codon) of mRNA) that are useful in antisense therapy against cancer and viral disease.

The gene for therapy or prophylaxis may be linked to a promoter for expressing the gene for therapy or prophylaxis. The promoter for expressing a gene for treatment or prevention is not particularly limited, but examples thereof can include a cytomegalovirus-derived promoter (including an enhancer as desired), an SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, and a phosphoglycerate kinase (PGK) promoter. The gene for treatment or prevention and the promoter for expressing the gene are preferably flanked by the ITR sequences.

In the present invention, a virus helper gene derived from an adenovirus may be preferably introduced into cells. The virus helper gene is a non-adeno-associated virus gene for enabling replication and packaging of an adeno-associated virus. As the virus helper gene, a gene derived from a virus of another species other than the adeno-associated virus is used. Specific examples of the virus helper gene include a virus helper gene derived from an adenovirus or a herpesvirus, and the virus helper gene is preferably derived from an adenovirus.

Examples of the virus helper gene derived from adenovirus can include E1A, E1B, E2A, E4, and VA-RNA. In the host cell having all or a part of the E1 region, the region of the adenovirus genome required for replicating the AAV genome and packaging of the AAV genome into the capsid to form a virus virion is the E2A region, the E4 region, and the VA1 RNA region. For the function by the E4 region, the 34 kDa E4 protein encoded by the open reading frame 6 (E4ORF6) of the E4 region is required for replicating the AAV. Preferably, the virus helper gene is an E2 gene, an E4 gene, or a VA-RNA gene. The VA-RNA gene is preferably a VA-RNAI gene.

The adenovirus-derived virus helper gene (such as an E1A, an E1B, an E2A, an E4, and a VA-RNA) may be a wild-type gene, but a gene in which modifications such as substitution, deletion, insertion, or addition of a base have been made to the wild-type gene may be used as long as it exhibits an inherent function.

In a case of introducing a virus helper gene into a cell, a vector containing the virus helper gene can be introduced into the cell.

As the vector containing a virus helper gene, for example, a plasmid, a virus-derived sequence, an artificially designed nucleic acid, or the like can be used, and a plasmid is preferable.

It is preferable that the virus helper gene is under the control of a promoter.

The specific examples of the promoter are not particularly limited, but can include a cytomegalovirus-derived promoter (CMV promoter) (including an enhancer as desired), an SV40 early promoter, a human elongation factor-1α (EF-1α) promoter, a human ubiquitin C promoter, a retrovirus Rous sarcoma virus (RSV) LTR promoter, a dihydrofolate reductase promoter, a β-actin promoter, and a phosphoglycerate kinase (PGK) promoter. The promoter may be under the control of a promoter capable of regulating expression. The promoter capable of regulating expression may be a Tet-on/off system which is a promoter capable of regulating expression by tetracycline, or a Tet-on system.

In the present invention, a protein can also be produced as a useful substance. The protein is preferably a recombinant protein. Examples of the protein include a recombinant polypeptide chain, a recombinant secreted polypeptide chain, an antigen-binding protein, an antibody (for example, a human antibody, a humanized antibody, a chimeric antibody, a mouse antibody, or a bispecific antibody), an Fc fusion protein, a fragmented immunoglobulin, and a single-chain antibody (scFv).

The protein is preferably an antibody, and more preferably a human antibody, a humanized antibody, a chimeric antibody, or a mouse antibody. Examples of the fragmented immunoglobulin include Fab, F(ab')₂, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine.

The antibody is not particularly limited; however, examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

In the present invention, a useful substance can be produced by culturing a cell into which a nucleic acid has been introduced.

The culture of cells can be performed under normal conditions for cell culture.

The culture temperature of the cells is not particularly limited, and the culture of the cells are performed at a temperature at which the cells can survive. The culture temperature is generally 25°C to 45°C, preferably 30°C to 42°C, more preferably 35°C to 40°C, and 37°C as an example.

The CO₂ concentration is generally 3 to 10% CO₂, preferably 5 to 10% CO₂, and 8% CO₂ as an example.

The period for producing a useful substance such as a virus is preferably 24 hours or more and 30 days or less, more preferably 24 hours or more and 20 days or less, still more preferably 24 hours or more and 10 days or less, even still more preferably 24 hours or more and 7 days or less, and particularly preferably 24 hours or more and 72 hours or less.

As the culture, batch culture, fed-batch culture, perfusion culture, or shaking culture can be performed. It is preferable that at least a part of the virus production step is perfusion culture.

The culture container is not particularly limited, and may be, for example, any one of a flask or a bioreactor.

The culture scale is not particularly limited, and the cells can be cultured in a culture medium of any volume, for example, the cells can be cultured in a culture medium of 1 mL to 2,500 L, preferably 1 L to 2,300 L, more preferably 50 L to 2,200 L, and particularly preferably 300 L to 2,100 L.

The culture may be carried out while stirring by shaking. The stirring speed in the case of stirring by shaking is generally 50 rpm to 200 rpm, and preferably 80 to 150 rpm.

The stirring culture may be rotary stirring culture using a propeller or the like in a reactor. The stirring speed in the case of stirring by rotating is generally 50 rpm to 200 rpm, and preferably 80 to 150 rpm. In addition, stirring may be performed by wave-type shaking stirring or vertical movement of the stirring blade, but the stirring is not particularly limited.

Examples of the culture medium include, but are not limited to, a BalanCD (registered trademark) culture medium, Expi293 Expression Medium (Thermo Fisher Scientific, A1435101), a Dulbecco's modified Eagle's medium (DMEM) containing 10% (vol/vol) fetal bovine serum (FBS), Gibco (trademark) Viral Production Medium (Thermo Fisher Scientific, A4817901), a serum-free UltraCULTURE (trademark) culture medium (Lonza), a HuMEC basal serum-free culture medium, a KNOCKOUT (trademark) CTS (trademark) XenoFREE ESC/iPSC culture medium, a STEMPRO (trademark)-34 SFM culture medium, a STEMPRO (trademark) NSC culture medium, an ESSENTIAL (trademark)-8 culture medium, a culture medium 254, a culture medium 106, a culture medium 131, a culture medium 154, a culture medium 171, a culture medium 200, a culture medium 231, a HeptoZYME-SFM, a human endothelial-SFM, a Gibco (registered trademark) FREESTYLE (trademark) 293 expression culture medium, a culture medium 154CF/PRF, a culture medium 154C, a culture medium 154 CF, a culture medium 200PRF, a culture medium 131, an Essential (trademark)-6 culture medium, a STEMPRO (trademark)-34 culture medium, a Gibco (registered trademark) Astrocyte culture medium, an AIM V (registered trademark) culture medium CTS (trademark), an AMINOMAX (trademark) C-100 basal culture medium, an AMINOMAX (trademark)-II complete culture medium, a CD FORTICHO (trademark) culture medium, a CD CHO AGT culture medium, a CHO-S-SFM culture medium, a Gibco (registered trademark) FREESTYLE (trademark) CHO expression culture medium, a CD OPTICHO (trademark) culture medium, a CD CHO culture medium, a CD DG44 culture medium, a SF-900 (trademark) culture medium, an EXPI293 (trademark) expression culture medium, an LHC basal culture medium, an LHC-8 culture medium, a 293 SFM culture medium, a CD 293 culture medium, an AEM growth culture medium, a PER.C6 (registered trademark) cell culture medium, an AIM V (registered trademark) culture medium, an EXPILIFE (registered trademark) culture medium, a keratinocyte SFM culture medium, an LHC culture medium, an LHC-8 culture medium, an LHC-9 culture medium, and any derivatives or modifications thereof. In a certain specific non-limiting embodiment, the high-density culture medium may be a CD FORTICHO (trademark) culture medium, a CD CHO AGT culture medium, a CHO-S-SFM culture medium, a GIBCO (registered trademark) FREESTYLE (trademark) CHO expression culture medium, a CD OPTICHO (trademark) culture medium, a CD CHO culture medium, a CD DG44 culture medium, a GIBCO (registered trademark) FREESTYLE (trademark) 293 expression culture medium, an EXPI293 (trademark) expression culture medium, an LV-MAX (trademark) production culture medium, a FREESTYLE (trademark) F17 expression culture medium, a DYNAMIS (trademark) culture medium, a similar culture medium, or a modified form thereof. The culture medium may be supplemented with 1x GlutaMAX (TM) (manufactured by Thermo Fisher Scientific, Inc.) or the like, as desired. Among these, a BalanCD (registered trademark) culture medium, Expi293 Expression Medium (Thermo Fisher Scientific, A1435101), a Dulbecco's modified Eagle's medium (DMEM) containing 10% (vol/vol) fetal bovine serum (FBS), Gibco (trademark) Viral Production Medium (Thermo Fisher Scientific, A4817901), or FREESTYLE (trademark) F17 expression culture medium is particularly preferable.

In a case where the useful substance produced in the present invention is a virus, the titer of the produced virus can be measured by a conventional method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding MgCl₂ and Benzonase to the collected supernatant to react. It is possible to measure the titer of a target virus by performing ddPCR (Droplet Digital PCR) using the sample containing the target virus obtained above as a ddPCR sample.

According to the present invention, there is provided a composition containing a cationic polymer and a nucleic acid, in which a concentration of the nucleic acid is 100 to 1,000 µg/mL, and an electrical conductivity is 1.0 to 7.0 mS/cm. The preferred ranges of the cationic polymer, the nucleic acid, the concentration of the nucleic acid, and the electrical conductivity are as described above in the present specification. The above-described composition is useful as a composition for gene introduction.

According to the present invention, there is further provided a cell culture product containing the above-described composition and cells having a cell density of 1 × 10⁵ cells/mL to 5 × 10⁸ cells/mL. The preferred ranges of the cell density and the cells are as described above in the present specification.

The present invention will be more specifically described using the following examples; however, it is not limited by the examples.

### Examples

### <Cell culture solution>

HEK-293 cells (Expi293, A14527, manufactured by Thermo Fisher Scientific, Inc.) were cultured in a BalanCD medium (manufactured by Fujifilm Irvine Scientific, Inc.) supplemented with 1x GlutaMAX (TM) (manufactured by Thermo Fisher Scientific, Inc.).

The cells were cultured in a shaking flask (manufactured by Corning Inc.) or a bioreactor. In the shaking flask, the cells were cultured in a 37°C incubator with stirring at 120 rpm in a humidified atmosphere of 8% CO₂. As a pre-culture, cells were seeded at 0.5 × 10⁶ cells/mL, and in a case where the cell density reached approximately 2 × 10⁶ cells/mL, gene introduction was performed by adding a nucleic acid complex.

### <Nucleic acid>

Three plasmid DNAs were used to produce an adeno-associated virus vector (rAAV): 1) a plasmid containing an introduction target gene in which ITRs are adjacent, 2) a packaging plasmid containing rep and cap genes, and 3) adenovirus helper plasmids E2, E4, and VARNA genes containing adenoviruses. In all plasmids, AAVpro Packaging Plasmid (AAV5, 6650, manufactured by Takara Bio Inc.) was used. For 1), Enhanced Green Fluorescent Protein (EGFP) was inserted downstream of the CMV promoter as a target gene and used.

### <Cationic polymer>

Linear polyethyleneimine (PEI) "PEI pro" having a molecular weight of 25 kDa (manufactured by Polyplus) was used as a gene introduction reagent.

### <Transfection>

The total amount of the plasmid DNA used for transfection was 0.5 µg per 1 million cells.

A PEI/DNA complex was prepared at a ratio between PEI and plasmid DNA of 1:1, 2:1, or 3:1, and incubated at room temperature for 15 minutes, and the plasmid DNA/PEI complex was added to the cell culture solution. As a result of the preliminary examination, a case where the mixing ratio between PEI and plasmid DNA was 2:1 was preferable from the viewpoint of gene introduction efficiency, thereby the following examples describe a case where the mixing ratio was 2:1.

In this case, the process of mixing the plasmid DNA with PEI to form a complex is described below, as compared with the standard conditions.

### Standard conditions (reference example):

The plasmid and PEI (solvent: BalanCD culture medium, electrical conductivity of 10.1 mS/cm) were mixed, and prepared such that the final concentration of the plasmid was 13 µg/mL and the final concentration of PEI was 26 µg/mL, and transfection into 2 × 10⁶ cells/mL of HEK cells was performed.

### High concentration conditions (present invention):

The plasmid and PEI (an electric conductivity-adjusting solvent in which BalanCD culture medium and 10 mM HEPES were mixed at a ratio of 3:7, 4:6, 5:5, and 6:4 was used as a solvent and an electrical conductivity of a solvent was each 3.7, 4.8, 5.6, 6.8 mS/cm before mixing the plasmid and PEI) were mixed, the final concentration of the plasmid was adjusted to 130 µg/mL, the final concentration of PEI was adjusted to 260 µg/mL, and transfection into 2 × 10⁷ cells/mL of HEK cells was performed.

* The electrical conductivity-adjusting solvent was prepared by mixing BalanCD culture medium and 10 mmol/L HEPES (Thermo Fisher Scientific) at a ratio of 4:6.

The sample containing cells and a culture medium for a cell culture solution was collected 24 hours after transfection for measuring the number of cells and the gene introduction efficiency, and was recovered 72 hours after transfection for evaluating the production amount of AAV.

### [Electrical conductivity]

Using an electrical conductivity meter, the electrode of the electrical conductivity meter was inserted into each sample in which the temperature was controlled to 25°C, and the electrical conductivity was measured.

### [Measurement of average particle diameter of complex]

The average particle diameter (standard of scattering intensity) of the complex was measured at 25°C using a dynamic light scattering method. The analysis method was a cumulant method, and as the viscosity and the refractive index, values measured with a viscometer and a refractometer were each used.

### [Cell density]

The cell density was determined using Vi-cell (trademark) XR (manufactured by Beckman Coulter, Inc.).

### [Gene introduction efficiency]

As the gene introduction efficiency, the proportion of EGFP-expressing cells 24 hours after the gene introduction operation was evaluated by flow cytometry (Attune Flow Cytometer, manufactured by Thermo Fisher Scientific, Inc.).

To compare the gene introduction efficiency with the conventional method, the evaluation was performed as a relative value with respect to the proportion of EGFP-expressing cells in the conventional method. In the reference example and the present invention, the ratio of the three plasmid DNAs was set to 1:1:1 as molar ratio of 3 types of plasmids.

### [Measurement of AAV titer]

An HEK cell culture solution that had been subjected to culture for 72 hours after gene introduction under the conditions of 37°C and 8% CO₂ was recovered. Thereafter, Triton X (manufactured by Thermo Fisher Scientific, Inc.) at a final concentration of 0.1%, MgCl₂ at a final concentration of 2 mmol/L, and 25 U/mL Benzonase (manufactured by Merck KGaA) were added thereto, and the mixture was reacted at 37°C for 2 hours to digest the gDNA (genomic DNA) and the residual plasmid. The sample after the reaction was incubated at 95°C for 15 minutes, 70°C for 3 minutes, 40°C for 3 minutes, and 20°C for 3 minutes in this order, to inactivate Benzonase, and the reactant was used as a ddPCR (Droplet Digital PCR) sample. The ddPCR sample was diluted 50-fold with a TE buffer (pH 8.0, containing 0.05% Pluronic F-68 and 10 µg/mL bovine thymus DNA). 10 µL of ddPCRtm Supermix for Probes (no dUTP) (Bio-Rad Laboratories, Inc.), 2 µL of diluted ddPCR sample, primers (seq1, seq2, final concentration of 900 nmol/L) and probe (seq3, final concentration of 250 nmol/L, FAM (fluorescein amide) was added as a fluorescent probe), nuclease free water (Thermo Fisher Scientific, 10977015) was mixed with each other in a tube on ice, the total liquid volume was adjusted to 22 µL, and ddPCR was performed.

In ddPCR, droplets were formed from the prepared solution using a droplet forming apparatus, and subsequently incubated at 95°C for 10 minutes, 94°C for 30 seconds, and 55°C in this order for 40 cycles, and then incubated at 98°C for 10 minutes. Measurement was performed using a droplet reader, and the AAV genomic titer (vg/mL) was calculated.
seq1 GGAACCCCTAGTGATGGAGTT (SEQ ID NO: 1)
seq2 CGGCCTCAGTGAGCGA (SEQ ID NO: 2)
seq3 CACTCCCTCTCTGCGCGCTCG (SEQ ID NO: 3)

Table 1 shows the electrical conductivity of the complex solution, the average particle diameter of the complex, and the gene introduction efficiency (relative value) in a case where the complex solution is transfected into cells at 2 × 10⁶ cells/mL.

Table 2 shows the electrical conductivity of the complex solution, the average particle diameter of the complex, the gene introduction efficiency (relative value), and an AAV5 genome titer (relative value) in a case where the complex solution is transfected into cells at 2 × 10⁷ cells/mL.

As shown in Tables 1 and 2, by controlling the formation of the complex in a solvent in which the electrical conductivity is defined, the introduction efficiency can be maintained even under the condition of forming a high-concentration complex.

As shown in Table 2, by controlling the formation of the complex in a solvent in which the electrical conductivity is defined and performing transfection, it is possible to achieve an AAV titer equal to or higher than that in the conventional method.

**[Table 1]**

| Transfection into cells at 2 × 10⁶ cells/mL | Electrical conductivity (mS/cm) | | Average particle diameter of complex (nm) | gene introduction efficiency (relative value) |
|---|---|---|---|---|
| | Only solvent | After addition of plasmid and PEI | | |
| Standard conditions (concentration of introduced nucleic acid: 13 µg/mL) | 10.5 | 10.1 | 801 | 1.00 |
| High concentration nucleic acid conditions (concentration of introduced nucleic acid: 130 µg/mL) | 0.2 | 0.2 | 115 | 0.04 |
| | 4.8 | 3.2 | 615 | 0.91 |
| | 5.6 | 3.9 | 912 | 0.94 |
| | 6.8 | 4.7 | 1,156 | 0.71 |
| | 10.5 | 7.6 | 1,641 | 0.44 |

**[Table 2]**

| Transfection into cells at 2 × 10⁷ cells/mL | Electrical conductivity (mS/cm) | | Average particle diameter of complex (nm) | gene introduction efficiency (relative value) | AAV5 genome titer (relative value) |
|---|---|---|---|---|---|
| | Only solvent | After addition of plasmid and PEI | | | |
| Standard conditions (concentration of introduced nucleic acid: 13 µg/mL) | 10.5 | 10.1 | 801 | 1.00 | 1.00 |
| High concentration nucleic acid conditions (concentration of introduced nucleic acid: 130 µg/mL) | 3.7 | 2.4 | 317 | 0.71 | 1.09 |
| | 4.8 | 3.2 | 615 | 1.03 | 1.99 |
| | 5.6 | 3.9 | 912 | 0.72 | 1.48 |
| | 10.5 | 7.6 | 1,641 | 0.29 | 0.34 |

| | | | | | |
|---|---|---|---|---|---|
| [Sequence list] International application 22F00838W1JP23029939_2.xml based on International Patent Cooperation Treaty | | | | | |

## Claims

1. A method of introducing a nucleic acid into a cell, the method comprising:
(a) a complex formation step of mixing a cationic polymer with a nucleic acid to obtain a solution of a complex of the cationic polymer and the nucleic acid, in which in the complex formation step, a concentration of the nucleic acid is 100 to 1,000 µg/mL and an electrical conductivity of the complex solution after the mixing is 1.0 to 7.0 mS/cm; and
(b) a transfection step of adding the complex solution obtained in the step (a) to a cell suspension.

2. The method according to claim 1,
wherein a period in which the electrical conductivity is 1.0 to 7.0 mS/cm is any one of 0 minutes to 10 minutes after a start of mixing the cationic polymer with the nucleic acid.

3. The method according to claim 1 or 2,
wherein the concentration of the nucleic acid is 110 µg/mL to 300 µg/mL.

4. The method according to claim 1 or 2,
wherein a mass ratio between the cationic polymer and the nucleic acid is 8:1 to 1:1.

5. The method according to claim 1 or 2,
wherein a cell density of the cell suspension in a case of being brought into contact with the complex solution is 1 × 10⁵ cells/mL to 5 × 10⁸ cells/mL.

6. The method according to claim 1 or 2,
wherein a cell density of the cell suspension in a case of being brought into contact with the complex solution is 5 × 10⁶ cells/mL to 3 × 10⁷ cells/mL.

7. The method according to claim 1 or 2,
wherein a total amount of the nucleic acid used for forming the complex is 0.1 to 1 µg per 1 million cells.

8. The method according to claim 1 or 2,
wherein the electrical conductivity of the complex solution is 3.0 to 5.0 mS/cm.

9. The method according to claim 1 or 2,
wherein an average particle diameter of the complex is 200 nm to 1,200 nm.

10. The method according to claim 1 or 2,
wherein the complex formation step (a) includes, before the step (b), mixing the cationic polymer with the nucleic acid and then allowing the solution of the complex to stand for 10 seconds to 4 hours.

11. The method according to claim 1 or 2,
wherein a volume ratio between the complex solution and the cell suspension is 1:2 to 1:500.

12. The method according to claim 1 or 2,
wherein the cationic polymer is a polyamine.

13. The method according to claim 1 or 2,
wherein the cationic polymer is polyethyleneimine.

14. The method according to claim 1 or 2,
wherein the nucleic acid is a plasmid.

15. The method according to claim 1 or 2,
wherein the cell is an animal cell.

16. The method according to claim 1 or 2,
wherein the cell is an HEK cell.

17. A method of producing a cell into which a nucleic acid has been introduced, the method comprising:
the method according to claim 1 or 2.

18. A method of producing a useful substance, comprising:
a step of introducing a nucleic acid into a cell by the method according to claim 1 or 2; and
a step of culturing the cell to produce a useful substance.

19. The method according to claim 18,
wherein the useful substance is a virus vector.

20. The method according to claim 19,
wherein the virus vector is an active pharmaceutical ingredient.

21. A composition comprising:
a cationic polymer; and
a nucleic acid,
wherein a concentration of the nucleic acid is 100 to 1,000 µg/mL, and
an electrical conductivity is 1.0 to 7.0 mS/cm.

22. The composition according to claim 21,
wherein the composition is a composition for gene introduction.

23. A cell culture product comprising:
the composition according to claim 21 or 22; and
cells having a cell density of 1 × 10⁵ cells/mL to 5 × 10⁸ cells/mL.
